# EUROPÄISCHE PATENTSCHRIFT

(11) **EP 1 002 516 B1**
(45) Veröffentlichungstag und Bekanntmachung des Hinweises auf die Patenterteilung: **24.09.2003**
(21) Anmeldenummer: 99116647.1
(22) Anmeldetag: 26.08.1999
(51) Int. Cl.: A61K 7/13

(54) **Haarfärbemittel mit einem Gehalt an Indanderivaten**
Hair dyes containing indan derivatives
Compositions tinctoriales pour cheveux renfermant des dérivés d'indane

(30) Priorität: 13.11.1998 DE 19852337
(43) Veröffentlichungstag der Anmeldung: 24.05.2000
(73) Patentinhaber: Wella Aktiengesellschaft, 64295 Darmstadt (DE)
(72) Erfinder: Chassot, Laurent, Dr., 1724 Praroman (CH); Descloux, Laurence, 1756 Lovens (CH)

(56) Entgegenhaltungen:
- DE-A- 3 009 833
- DE-A- 4 215 654
- DE-A- 4 317 855
- DE-A- 4 335 628
- PATENT ABSTRACTS OF JAPAN vol. 13, no. 182, 27. April 1989 (1989-04-27) & JP 01 009968 A (SUMITOMO), 13. Januar 1989 (1989-01-13)
- CHEMICAL ABSTRACTS, vol. 126, no. 16, 21. April 1997 (1997-04-21) Columbus, Ohio, US; abstract no. 213337x, K. BELLI ET AL : "Novel monoazo disperse dyes derived from 3,5-dibromo-aminobentaldehyde as diazo component" Seite 693; XP002133260 & COLOURAGE, Bd. 44, Nr. 1, 1997, Seiten 49-52,

## Beschreibung

Gegenstand der vorliegenden Erfindung sind Mittel zur Färbung von Haaren, enthaltend 1,3-Bis(dicyan-methylen)indanfarbstoffe, sowie die Verwendung von 1,3-Bis(dicyan-methylen)indanfarbstoffen zur Färbung von Fasern.

Für die farbverändemde Behandlung von keratinhaltigen Fasern werden in der Regel zwei Färbeverfahren angewendet. Im ersten Verfahren wird die Färbung mit sogenannten oxidativen oder permanenten Färbemitteln unter Verwendung einer Mischung aus verschiedenen Entwicklersubstanzen und Kupplersubstanzen und eines Oxidationsmittels erzeugt. Bei Bedarf können bei diesem Verfahren zur Abrundung des Färbeergebnisses oder zur Erzeugung von besonderen Farbeffekten sogenannte direktziehende (nicht-oxidative) Farbstoffe zugesetzt werden. Das zweite Verfahren bedient sich ausschließlich direktziehender Farbstoffe, die in einer geeigneten Trägermasse auf die Fasern aufgebracht werden. Dieses Verfahren ist einfach anzuwenden, ausgesprochen mild und zeichnet sich durch eine geringe Schädigung der Keratinfaser aus. An die hierbei verwendeten direktziehenden Farbstoffe werden eine Vielzahl von Anforderungen gestellt. So müssen sie in toxikologischer und dermatologischer Hinsicht unbedenklich sein und die Erzielung von Färbungen in der gewünschten Intensität ermöglichen, was unter anderem auch eine ausreichende Wasserlöslichkeit voraussetzt. Außerdem wird für die erzielten Färbungen eine gute Lichtechtheit, Säureechtheit und Reibechtheit gefordert.

Für ein direktziehendes (nicht-oxidatives) Färbemittel für Keratinfasem wird in der Regel eine Kombination von verschiedenen nicht-oxidativen Farbstoffen benötigt. Da die Auswahl an roten und blauen Farbstoffen, die in Färbemittel für Keratinfasem, insbesondere menschlichen Haaren, eingesetzt werden können, beschränkt ist, besteht ein dringender Bedarf an der Erweiterung der nutzbaren Farbstoffpalette.

Aus der JP 64-9968 A sind 1,3-Bis(dicyan-methylen)indane sowie ein Verfahren zu deren Herstellung bekannt.

Es wurde nun überraschend gefunden, daß bestimmte Indanderivate Fasern, insbesondere Keratinfasem wie zum Beispel Haare, intensiv blau färben.

Gegenstand der vorliegenden Erfindung ist deshalb ein Mittel zur Färbung von Haaren, welches dadurch gekennzeichnet ist, daß es (a) mindestens ein Indanderivat der Formel (1) oder dessen physiologisch verträgliches, wasserlösliches Salz, 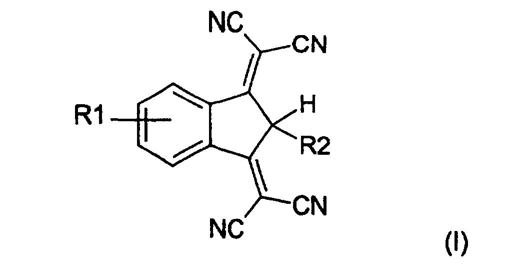 wobei die Reste R1und R2 unhabängig voneinander die folgende Bedeutung haben:
R1 = Wasserstoff, einem Halogenatom, einer Cyanogruppe, einer Hydroxygruppe, einer C₁-C₄-Alkoxygruppe, einer C₁-C₆-Alkylgruppe, einer Nitrogruppe, einer Aminogruppe, einer Alkylaminogruppe, einer Dialkylaminogruppe, einer -C(O)H-Gruppe, einer -COOR3 -Gruppe, einer SO₂R3 oder einer -C(O)CH₃-Gruppe (mit R3 jeweils gleich Wasserstoff, einer Phenylgruppe oder einer C₁-C₆-Alkygruppe);
R2 gleich Wasserstoff, einer C₁-C₆-Alkygruppe oder einer Phenylgruppe; sowie (b) für Haarfärbemittel übliche, kosmetisch verträgliche Zusätze enthält.

Bevorzugte Indanderivate gemäß der allgemeinen Formel (I) sind solche mit R₁ = Wasserstoff oder -CH₃ und R2 = Methyl, Phenyl oder insbesondere Wasserstoff.

Als physiologisch verträgliche Salze der Verbindungen der Formel (I) sind insbesondere Alkalisalze und Ammoniumsalze, beispielsweise die Ammoniumsalze, Triethylammoniumsalze, Natriumsalze, Kaliumsalze, NN-Methylmorpholiniumsalze, Monoethanolammoniumsalze, Diethanolammoniumsalze und Triethanolammoniumsalze, zu nennen.

Die Verbindungen der Formel (I) werden in dem erfindungsgemäßen Mittel in einer Gesamtmenge von etwa 0,01 bis 10 Gewichtsprozent, insbesondere 0,01 bis 5 Gewichtsprozent, eingesetzt.

Die Verbindungen der Formel (I) färben keratinisches Material ohne den Zusatz weiterer Farbstoffe in intensiv blauen Farbtönen.
Zur Erzielung weiterer Farbnuancen können zusätzlich übliche direktziehende Farbstoffe, beispielsweise Nitrofarbstoffe, Azofarbstoffe oder Chinonfarbstoffe, sowie basische, saure oder neutrale Farbstoffe, alleine oder im Gemisch miteinander, zugesetzt werden.

Beispiele für geeignete Nitrofarbstoffe sind 1,4-Bis[(2-hydroxyethyl)-amino]-2-nitrobenzol, 1-(2-Hydroxyethyl)amino-2-nitro-4-[di(2-hydroxyethyl)amino]-benzol (HC Blue No. 2), 1-Amino-3-methyl-4-[(2-hydroxyethyl)amino]-6-nitrobenzol (HC Violet No. 1), 4-[Ethyl-(2-hydroxyethyl)amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxypropyl)amino]-4-[methyl-(2-hydroxyethyl)amino]-2-nitrobenzol (HC Blue No. 10), 1-[(2,3-Dihydroxypropyl)amino]-4-[ethyl-(2-hydroxyethyl)amino]-2-nitrobenzolhydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxypropyl)amino]-2-nitrobenzol (HC Blue No. 6), 2-((4-Amino-2-nitrophenyl)amino)-5-dimethylaminobenzoesäure (HC Blue No. 13), 1-(2-Aminoethylamino)-4-[di(2-hydroxyethyl)amino]-2-nitrobenzol, 1-Amino-4-[(2-hydroxyethyl)amino]-2-nitrobenzol (HC Red No. 7), 2-Amino-4,6-dinitro-phenol, 4-Amino-2-nitrodiphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-((2-Hydroxyethyl)methylamino)-1-(methylamino)-2-nitrobenzol, 1-Amino-4-((2,3-dihydroxypropyl)amino)-5-methyl-2-nitrobenzol, 1-Amino-4-(methylamino)-2-nitrobenzol, 4-Amino-2-nitro-1-((prop-2-en-1-yl)amino)-benzol, 4-Amino-3-nitrophenol, 4-[(2-Hydroxyethyl)amino]-3-nitrophenol, 1-[(2-Aminoethyl)amino]-4-(2-hydroxyethoxy)-2-nitrobenzol (HC Orange No. 2), 4-(2,3-Dihydroxypropoxy)-1-[(2-hydroxyethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino] -2-nitrobenzol (HC Red No. 11), 2-[(2-Hydroxyethyl)amino]-4,6-dinitro-phenol, 4-Ethylamino-3-nitrobenzoesäure, 2-[(4-Amino-2-nitrophenyl)amino]-benzoesäure, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitropyridin, 6-Amino-3-((2-hydroxyethyl)amino)-2-nitropyridin, 3-Amino-6-((2-hydroxyethyl)amino)-2-nitropyridin, 3-Amino-6-(ethylamino)-2-nitropyridin, 3-((2-Hydroxyethyl)amino)-6-(methylamino)-2-nitropyridin, 3-Amino-6-(methylamino)-2-nitropyridin, 6-(Ethylamino)-3-((2-hydroxyethyl)amino)-2-nitropyridin, 1,2,3,4-Tetrahydro-6-nitrochinoxalin, 7-Amino-3,4-dihydro-6-nitro-2H-1,4-benzoxazin (HC Red No. 14), 1-Amino-2-[(2-hydroxyethyl)-amino]-5-nitrobenzol (HC Yellow No. 5), 1-(2-Hydroxyethoxy)-2-[(2-hydroxyethyl)amino]-5-nitrobenzol (HC Yellow No. 4), 1-[(2-Hydroxyethyl)-amino]-2-nitrobenzol (HC Yellow No. 2), 2-(Di(2-hydroxyethyl)amino)-5-nitrophenol, 2-[(2-Hydroxyethyl)amino]-1-methoxy-5-nitrobenzol, 2-Amino-3-nitrophenol, 1-(2-Hydroxyethoxy)-3-methylamino-4-nitrobenzol, 2,3-(Dihydroxypropoxy)-3-methylamino-4-nitrobenzol, 2-[(2-Hydroxyethyl)-amino]-5-nitrophenol (HC Yellow No. 11), 3-[(2-Aminoethyl)amino]-1-methoxy-4-nitrobenzol-hydrochlorid (HC Yellow No.9), 1-[(2-Ureidoethyl)amino]-4-nitrobenzol, 4-[(2,3-Dihydroxypropyl)amino]-3-nitro-1-trifluormethyl-benzol (HC Yellow No. 6), 1-Chlor-2,4-bis[(2-hydroxyethyl)amino]-5-nitrobenzol (HC Yellow No. 10), 1-Amino-4-((2-aminoethyl)amino)-5-methyl-2-nitrobenzol, 4-[(2-Hydroxyethyl)amino]-3-nitro-1-methylbenzol, 1-Chlor-4-[(2-hydroxyethyl)amino]-3-nitrobenzol (HC Yellow No. 12), 4-[(2-Hydroxyethyl)amino]-3-nitro-1-trifluormethyl-benzol (HC Yellow No. 13), 4-[(2-Hydroxyethyl)amino]-3-nitro-benzonitril (HC Yellow No. 14), 4-[(2-Hydroxyethyl)amino]-3-nitro-benzamid (HC Yellow No. 15), 3-((2-Hydroxyethyl)amino)-4-methyl-1-nitrobenzol und 4-Chlor-3-((2-hydroxyethyl)amino) -1-nitrobenzol.

Beispiele für geeignete Chinonfarbstoffe sind 1,4-Di[(2,3-dihydroxypropyl)-amino]-9,10-anthrachinon, 1-((2-Hydroxyethyl)arnino]-4-methylamino-9,10-anthrachinon (CI61505, Disperse Blue No. 3), 2-[(2-Aminoethyl)amino]-9,10-anthrachinon (HC Orange No. 5), 1-Hydroxy-4-[(4-methyl-2-sulfophenyl)amino]-9,10-anthrachinon, 1-[(3-Aminopropyl)amino]-4-methylamino-9,10-anthrachinon (HC Blue No. 8),1-[(3-Aminopropyl)-amino]-9,10-anthrachinon (HC Red No. 8), 1,4-Diamino-2-methoxy-9,10-anthrachinon (CI62015, Disperse Red No. 11, Solvent Violet No. 26), 1,4-Dihydroxy-5,8-bis[(2-hydroxyethyl)amino]-9,10-anthrachinon und (CI62500, Disperse Blue No. 7, Solvent Blue No. 69).

Beispiele für geeignete basische Farbstoffe sind 9-(Dimethylamino)-benzo[a]phenoxazin-7-ium-chlorid (CI51175; Basic Blue No. 6), Di[4-(diethylamino)phenyl][4-(ethylamino)naphthyl]carbenium-chlorid (CI42595; Basic Blue No. 7), Di-(4-(dimethylamino)phenyl)-(4-(methylphenylamino)naphthalin-1-yl)carbenium-chlorid (CI42563; Basic Blue No. 8), 3,7-Di(dimethylamino)phenothiazin-5-ium-chlorid (CI52015; Basic Blue No. 9), Di[4-(dimethylamino)phenyl][4-(phenylamino)naphthyl]carbeniumchlorid (CI44045; Basic Blue No. 26), 2-[(4-(Ethyl(2-hydroxyethyl)-amino)phenyl)azo]-6-methoxy-3-methyl-benzothiazolium-methylsulfat (CI11154; Basic Blue No. 41), 8-Amino-2-brom-5-hydroxy-4-imino-6-[(3-(trimethylammonio)phenyl)amino]-1(4H)-naphthalinon-chlorid (Cl56059; Basic Blue No. 99), Bis[4-(dimethylamino)phenyl][4-(methylamino)-phenyl]carbenium-chlorid (CI42535; Basic Violet No. 1), Tris[4-(dimethylamino)phenyl]carbenium-chlorid (CI42555; Basic Violet No. 3), 2-[3,6-(Diethylamino)dibenzopyranium-9-yl]-benzoesäure-chlorid (CI45170; Basic Violet No. 10), Di(4-aminophenyl)(4-amino-3-methylphenyl)carbenium-chlorid (CI42510; Basic Violet No. 14),

1,3-Bis((2,4-diamino-5-methylphenyl)azo]-3-methylbenzol (Cl2101;Basic Brown No. 4), 1-[(4-Aminophenyl)azo]-7-(trimethylammonio)-2-naphtholchlorid (Cl12250; Basic Brown No. 16), 1-[(4-Amino-2-nitrophenyl)azo]-7-(trimethylammonio)-2-naphthol-chlorid (Basic Brown No. 17), 1-[(4-Amino-3-nitrophenyl)azo]-7-(trimethylammonio)-2-naphthol-chlorid (Cl12251; Basic Brown No. 17), 3,7-Diamino-2,8-dimethyl-5-phenylphenazinium-chlorid (CI50240; Basic Red No. 2), 1,4-Dimethyl-5-[(4-(dimethylamino)phenyl)azo]-1,2,4-triazolium-chlorid (CI11055; Basic Red No. 22), 2-Hydroxy-1-[(2-methoxyphenyl)azo]-7-(trimethylammonio)-naphthalin-chlorid (CI12245; Basic Red No. 76), 2-[2-((2,4-Dimethoxyphenyl)amino)ethenyl]-1,3,3-trimethyl-3H-indol-1-ium-chlorid (C148055; Basic Yellow No. 11), 3-Methyl-1-phenyl-4-[(3-(trimethylammonio)phenyl)-azo]-pyrazol-5-on-chlorid (CI12719; Basic Yellow No. 57), Di(4-(dimethylamino)phenyl)iminomethan-hydrochlorid (CI41000; Basic Yellow No. 2), Bis[4-(diethylamino)phenyl]phenylcarbenium-hydrogensulfat(1:1) (CI42040; Basic Green No. 1) und Di(4-(dimethylamino)phenyl)-phenylmethanol (CI42000; Basic Green No. 4).

Beispiele für geeignete neutrale Azofarbstoffe sind 1-[Di(2-hydroxyethyl)-amino]-3-methyl-4-[(4-nitrophenyl)azo]-benzol (CI11210, Disperse Red No. 17), 4-[(4-Aminophenyl)azo]-1-[di(2-hydroxyethyl)amino]-3-methylbenzol (HC Yellow No. 7), 2,6-Diamino-3-[(pyridin-3-yl)azo]-pyridin und 2-((4-(Acetylamino)phenyl)azo)-4-methylphenol (CI11855; Disperse Yellow No. 3).

Beispiele für geeignete saure Farbstoffe sind 6-Hydroxy-5-[(4-sulfophenyl)azo]-2-naphthalinsulfonsäure-dinatriumsalz (Cl15985; Food Yellow No. 3; FD&C Yellow No. 6), 2,4-Dinitro-1-naphthol-7-sulfonsäuredinatriumsalz (CI10316; Acid Yellow No. 1; Food Yellow No. 1), 2-(lndan-1,3-dion-2-yl)chinolin-x,x-sulfonsäure (Gemisch aus Mono- und Disulfonsäure) (Cl47005;D&C Yellow No. 10; Food Yellow No. 13; Acid Yellow No. 3), 5-Hydroxy-1-(4-sulfophenyl)-4-[(4-sulfophenyl)-azo]pyrazol-3-carbonsäure-trinatriumsalz (CI19140; Food Yellow No. 4; Acid Yellow No. 23), 9-(2-Carboxyphenyl)-6-hydroxy-3H-xanthen-3-on (CI45350; Acid Yellow No. 73; D&C Yellow No. 8), 4-((4-Amino-3-sulfophenyl)azo)benzolsulfonsäure-dinatriumsalz (Cl13015, Acid Yellow No. 9),5-[(2,4-Dinitrophenyl)amino]-2-phenylamino-benzolsulfonsäurenatriumsalz (Cl10385; Acid Orange No. 3), 4-[(2,4-Dihydroxyphenyl)azo]-benzolsulfonsäure-mononatriumsalz (Cl14270; Acid Orange No. 6), 4-((2-Hydroxynaphth-1-yl)azo]-benzolsulfonsäure-natriumsalz (Cl15510; Acid Orange No. 7), 4-[(2,4-Dihydroxy-3-[(2,4-dimethylphenyl)azo]-phenyl)azo]-benzolsulfonsäure-natriumsalz (CI20170; Acid Orange No. 24), 4-Hydroxy-3-[(4-sulfonaphth-1-yl)azo]-1-naphthalin-sulfonsäuredinatriumsalz (Cl14720; Acid Red No. 14), 6-Hydroxy-5-[(4-sulfonaphth-1-yl)azo]-2,4-naphthalin-disulfonsäure-trinatriumsalz (Cl16255; Ponceau 4R; Acid Red No. 18), 3-Hydroxy-4-[(4-sulfonaphth-1-yl)azo)-2,7-naphthalin-disulfonsäure-trinatriumsalz (Cl16185; Acid Red No. 27), 8-Amino-1-hydroxy-2-(phenylazo)-3,6-naphthalin-disulfonsäuredinatriumsalz (Cl17200; Acid Red No. 33), 5-(Acetylamino)-4-hydroxy-3-[(2-methylphenyl)azo]-2,7-naphthalin-disulfonsäure-dinatriumsalz (CI18065; Acid Red No. 35), 2-(3-Hydroxy-2,4,5,7-tetraiod-dibenzopyran-6-on-9-yl)-benzoesäure-dinatriumsalz (CI45430;Acid Red No. 51), N-[6-(Diethylamino)-9-(2,4-disulfophenyl)-3H-xanthen-3-yliden]-N-ethylethanammonium-hydroxid, inneres Salz, Natriumsalz (CI45100; Acid Red No. 52), 8-[(4-(Phenylazo)phenyl)azo]-7-naphthol-1,3-disulfonsäuredinatriumsalz (CI27290; Acid Red No. 73), 2',4',5',7'-Tetrabrom-3',6'dihydroxyspiro[isobenzofuran-1(3H),9'-[9H]xanthen]-3-on-dinatriumsalz (CI45380; Acid Red No. 87), 2',4',5',7'-Tetrabrom-4,5,6,7-tetrachlor-3',6'dihydroxyspiro[isobenzofuran-1(3H),9'[9H]xanthen]-3-on-dinatriumsalz (CI45410; Acid Red No. 92), 3',6'-Dihydroxy-4',5'-diiodospiro-[isobenzofuran-1(3H),9'(9H)-xanthen]-3-on-dinatriumsalz (CI45425; Acid Red No. 95), 2-Hydroxy-3-((2-hydroxynaphth-1-yl)azo)-5-nitrobenzolsulfonsäure-mononatriumsalz (Cl15685; Acid Red No. 184), (2-Sulfophenyl)di[4-(ethyl((4-sulfophenyl)methyl)amino)phenyl]-carbenium-dinatriumsalz, betain (CI42090; Acid Blue No. 9; FD&C Blue No. 1), 1,4-Bis((2-sulfo-4-methylphenyl)amino]-9,10-anthrachinondinatriumsalz (Cl 61570; Acid Green No. 25), Bis[4-(dimethylamino)-phenyl]-(3,7-disulfo-2-hydroxynaphth-1-yl)-carbenium-inneres Salz, mononatriumsalz (CI44090; Food Green No. 4; Acid Green No. 50), Bis[4-(diethylamino)phenyl](2,4-disulfophenyl)carbenium-inneres salz, Natriumsalz (2:1) (CI42045; Food Blue No. 3; Acid Blue No. 1), Bis(4-(diethylamino)phenyl](5-hydroxy-2,4-disulfophenyl)carbeniuminneres salz, Calciumsalz (2:1) (C142051; Acid Blue No. 3), 1-Amino-4-(cyclohexylamino)-9,10-anthrachinon-2-sulfonsäure-natriumsalz (CI62045; Acid Blue No. 62), 2-(1,3-Dihydro-3-oxo-5-sulfo-2H-indol-2-yliden)-2,3-dihydro-3-oxo-1H-indol-5-sulfonsäure-dinatriumsalz (CI73015; Acid Blue No. 74), 9-(2-Carboxyphenyl)-3-[(2-methylphenyl)amino]-6-[(2-methyl-4-sulfophenyl)amino]xanthylium-inneres Salz, mononatriumsalz (CI45190; Acid Violet No. 9), 1-Hydroxy-4-[(4-methyl-2-sulfophenyl)amino]-9,10-anthrachinon-natriumsalz (CI60730; D&C Violett No. 2; Acid Violet No. 43), Bis[3-nitro-4-[(4-phenylamino)-3-sulfophenylamino]-phenyl]-sulfon (Cl10410; Acid Brown No. 13), 5-Amino-4-hydroxy-6-[(4-nitrophenyl)azo]-3-(phenylazo)-2,7-(naphthalindisulfonsäure-dinatriumsalz (CI20470; Acid Black No. 1), 3-Hydroxy-4-[(2-hydroxynaphth-1-yl)azo]-7-nitro-1-naphthalin-sulfonsäure-chromkomplex (3:2) (CI15711; Acid Black No. 52), 3-[(2,4-Dimethyl-5-sulfophenyl)azo]-4-hydroxy-1-naphthalin-sulfonsäure-dinatriumsalz (Cl14700; Food Red No. 1; Ponceau SX; FD&C Red No. 4), 4-(Acetylamino)-5-hydroxy-6-[(7-sulfo-4-[(4-sulfophenyl)azo]naphth-1-yl)azo]-1,7-naphthalindisulfonsäuretetranatriumsalz (CI28440; Food Black No. 1) und 3-Hydroxy-4-(3-methyl-5-oxo-1-phenyl-4,5-dihydro-1H-pyrazol-4-ylazo)-naphthalin-1-sulfonsäurenatriumsalz, Chrom-Komplex (Acid Red No. 195).

Beispiele für weitere geeignete Farbstoffe sind 4-((5-((2-Hydroxyethyl)-amino-1-methyl-1H-pyrazol-4-yl)imino)-4,5-dihydro-5-((2--hydroxyethyl)-imino)-1-methyl-1H-pyrazol-monosulfat, 5-Hydroxy-1,4-naphthochinon (Cl75500 Natural Brown No. 7), 2-Hydroxy-1,4-naphthochinon (CI75480, Natural Orange No. 6) und 1,2-Dihydro-2-(1,3-dihydro-3-oxo-2H-indol-2-yliden)-3H-indol-3-on (C173000).

Die genannten direktziehenden Farbstoffe können in einer Gesamtmenge von 0,01 bis 4 Gewichtsprozent enthalten sein.

Die Verbindungen der Formel (I) sind nicht nur für die Färbung von Keratinfasern, insbesondere menschlichen Haaren, geeignet, sondern sie können auch zum Färben von anderen Naturfasern, wie zum Beispiel Baumwolle, Jute, Sisal, Leinen oder Seide, modifizierten Naturfasern, wie zum Beispiel Regeneratcellulose, Nitrocellulose, Alkylcellulose oder Hydroxyalkylcellulose oder Acetylcellulose und synthetischen Fasern, wie zum Beispiel Polyamidfasern, Polyurethanfasern oder Polyesterfasern, verwendet werden.

Die Zubereitungsform des erfindungsgemäßen Haarfärbemittels kann beispielsweise eine Lösung, insbesondere eine wäßrige oder wäßrigalkoholische Lösung, eine Creme, ein Gel, eine tensidhaltige schäumende Lösung (Shampoo, Aerosol), eine Emulsion oder ein anderer für die Anwendung auf dem Haar geeigneter, wasserhaltiger Träger, sein. Die Zusammensetzung dieser Mittel stellt eine Mischung der Farbstoffkomponente mit den für solche Zubereitungen üblichen Zusätzen dar.

Übliche Zusätze in Lösungen, Crèmes, Emulsionen oder Gelen sind zum Beispiel Lösungsmittel wie Wasser, niedere aliphatische einwertige oder mehrwertige Alkohole, deren Ester und Ether, beispielsweise Alkanole, insbesondere mit 1 bis 4 C-Atomen, beispielsweise Ethanol, Propanol oder Isopropanol, Butanol, Isobutanol, zweiwertige und dreiwertige Alkohole, insbesondere solche mit 2 bis 6 C-Atomen, beispielsweise Ethylenglycol, Propylenglycol, 1,3-Propandiol, 1,4-Butandiol, 1,5-Pentandiol, 1,6-Hexandiol, 1,2,6-Hexantriol, Glycerin, Diethylenglycol, Dipropylenglycol, Polyalkylenglycole, wie Triethylenglycol, Polyethylenglycol, Tripropylenglycol, Polypropylenglycol, niedere Alkylether von mehrwertigen Alkoholen, wie Ethylenglycolmonomethylether oder Ethylenglycolmonoethylether oder Ethylenglycolmonopropylether oder Ethylenglycolmonobutylether, Diethylenglycolmonomethylether oder Diethylenglycolmonoethylether, Triethylenglycolmonomethylether oder Triethylenglycolmonoethylether, Ketone und Ketoalkohole, insbesondere solche mit 3 bis 7 C-Atomen, wie zum Beispiel Aceton, Methylethylketon, Diethylketon, Methylisobutylketon, Methylphenylketon, Cyclopentanon, Cyclohexanon und Diacetonalkohol, Ether, wie zum Beispiel Dibutylether, Tetrahydrofuran, Dioxan, Diisopropylether, Ester wie zum Beispiel Ethylformiat, Methylformiat, Methylacetat, Ethylacetat, Propylenacetat, Butylacetat, Phenylacetat, Ethylenglycolmonoethyletheracetat und Essigsäurehydroxyethylester, Amide wie zum Beispiel Dimethylformamid und Dimethylacetamid, N-Methylpyrrolidon, sowie Harnstoff, Tetramethylhamstoff und Thiodiglycol.

Weiterhin können in dem erfindungsgemäßen Haarfärbemittel Netzmittel oder Emulgatoren aus den Klassen der anionischen, kationischen, amphoteren, nichtionogenen oder zwitterionischen oberflächenaktiven Substanzen wie Fettalkoholsulfate, Alkansulfonate, Alkylbenzolsulfonate, Alkyltrimethylammoniumsalze, Alkylbetaine, α-Olefinsulfonate, oxethylierte Fettalkohole, oxethylierte Nonylphenole, Fettsäurealkanolamine, oxethylierte Fettsäureester, Fettalkoholpolyglycolethersulfate, Alkylpolyglucoside, Verdickungsmittel wie höhere Fettalkohole, Stärke, Cellulosederivate, Vaseline, Paraffinöl, Fettsäuren und andere Fettkomponenten in emulgierter Form, wasserlösliche polymere Verdickungsmittel wie natürliche Gummen, Guargummi, Xanthangummi, Johannisbrotkernmehl, Pektin, Dextran, Agar-Agar, Amylose, Amylopektin, Dextrine, Tone oder vollsynthetische Hydrokolloide wie Polyvinylalkohol, außerdem Pflegestoffe wie Lanolinderivate, Cholesterin, Pantothensäure, wasserlösliche kationische Polymere, Proteinderivate, Provitamine, Vitamine, Pflanzenextrakte, Zucker und Betain, Hilfsstoffe wie Feuchthaltemittel, Elektrolyte, Antioxidantien, Fettamide, Sequestrierungsmittel, filmbildende Agentien und Konservierungsmittel, enthalten sein. Neben Wasser kann auch auch ein wasserlösliches organisches Lösungsmittel oder ein Gemisch derartiger Lösungsmittel sowie ein Wasser/Lösungsmittel-Gemisch verwendet werden.

Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Menge von etwa 0,1 bis 5 Gewichtsprozent.

Je nach Zusammensetzung können die erfindungsgemäßen Färbemittel schwach sauer, neutral oder alkalisch reagieren, wobei die Einstellung von sauren pH-Werten vorzugsweise mit Zitronensäure, Milchsäure, Weinsäure oder Phosphorsäure erfolgt, während die Einstellung von alkalischen pH-Werten vorzugsweise mit Ammoniak erfolgt. Es können aber anstelle von Ammoniak auch andere organische Amine wie Monoethanolamin, Diethanolamin, Triethanolamin, N-Methyl-N-ethanolamin, N-Methyl N-Methyl-N,N-diethanolamin, 2-(2-Hydroxyethoxy)-ethanolamin, Di-2-(2-hydroxyethoxy)-ethanamin und Tri-2-(2-hydroxyethoxy)-ethanamin oder anorganische Basen wie Hydroxide, zum Beispiel Lithiumhydroxid, Natriumhydroxid und Kaliumhydroxid, oder Carbonate, beispielsweise Lithiumcarbonat, Natriumcarbonat oder Kaliumcarbonat, Verwendung finden. Vorzugsweise das erfindungsgemäße Mittel einen pH-Wert von 5 bis 8 auf.

Man läßt die Färbelösung bei 20 bis 50 °C etwa 10 bis 45 Minuten lang, vorzugsweise bei 40 °C 40 Minuten lang, auf das Haar einwirken, spült sodann das Haar mit Wasser aus und trocknet es. Gegebenenfalls wird im Anschluß an diese Spülung mit einem Shampoo gewaschen und/oder mit einer schwachen organischen Säure nachgespült. Anschließend wird das Haar getrocknet.

Das erfindungsgemäße Haarfärbemittel führt zu Haarfärbungen mit ausgezeichneten Echtheitseigenschaften, insbesondere was die Lichtechtheit, Waschechtheit und Reibechtheit anbetrifft. Bemerkenswert ist die große Farbintensität und die Farbreinheit der erzielbaren Färbungen. Schließlich ist mit Hilfe des erfindungsgemäßen Haarfärbemittels auch eine Anfärbung von ergrautem und chemisch nicht vorgeschädigtem Haar problemlos und mit sehr guter Deckkraft möglich. Die dabei erhaltenen Färbungen sind, unabhängig von der unterschiedlichen Struktur des Haares, gleichmäßig und sehr gut reproduzierbar.

Die Verbindungen der Formel (I) können durch Umsetzung der entsprechenden 1,3-Indandione mit Malonsäuredinitril dargestellt werden. Eine allgemeine Herstellungsmethode wird zum Beispiel von K.A. Bello, L. Cheng und J. Griffiths in J. Chem. Soc. Perkin Trans. II Seite 817 (1987) beschrieben.

Die nachfolgenden Beispiele sollen den Erfindungsgegenstand näher erläutern, ohne diesen jedoch darauf zu beschränken.

### Beispiele

### Beispiel 1: Synthese von 1,3-Bis (dicyanmethylen)indan

0,08 mol 1,3-Indandion und 0,28 mol Malondinitril werden bei Raumtemperatur in 200 ml Ethanol gerührt. Nach 15 Minuten werden 0,12 mol Natriumacetat-Trihydrat zugegeben. Das Reaktionsgemisch wird etwa 5 Stunden unter Rückfluß erhitzt. Nach beendeter Reaktion wird die Reaktionmischung abfiltriert, mit 400 ml Wasser versetzt und auf einen pH-Wert von 1 bis 2 mit Salzsäure eingestellt. Der Niederschlag wird abfiltriert, mit Wasser gewaschen und sodann getrocknet. Die Ausbeute betragt 85% der Theorie; das Produkt hat einen Schmelzpunkt von 224-228 °C

| CHN-Analyse: | | | |
|---|---|---|---|
| (C₁₅H₆N₄) | % C | % H | % N |
| berechnet | 74,37 | 2,50 | 23,13 |
| gefunden | 74,11 | 2,62 | 23,05 |

### Beispiel 2: Synthese von 2-Methyl-1,3-bis(dicyanmethylen)indan

0,04 mol 2-Methyl-1,3-indandion und 0,12 mol Malondinitril werden bei Raumtemperatur in 200 ml Ethanol gerührt. Nach 15 Minuten werden 0,6 mol Natriumacetat-Trihydrat zugegeben. Das Reaktionsgemisch wird etwa 5 Stunden unter Rückfluß erhitzt. Nach beendeter Reaktion wird die Reaktionmischung abfiltriert, mit 200 ml Wasser versetzt und auf einen pH-Wert von 1 bis 2 mit Salzsäure eingestellt. Der Niederschlag wird abfiltriert, mit Wasser gewaschen und sodann getrocknet.

### Beispiel 3: Synthese von 2-Phenyl-1,3-bis(dicyanmethylen)indan

0,04 mol 2-Phenyl-1,3-indandion und 0,12 mol Malondinitril werden bei Raumtemperatur in 200 ml Ethanol gerührt. Nach 15 Minuten werden 0,6 mol Natriumacetat-Trihydrat zugegeben. Das Reaktionsgemisch wird etwa 5 Stunden unter Rückfluß erhitzt. Nach beendeter Reaktion wird die Reaktionmischung abfiltriert, mit 200 ml Wasser versetzt und auf einen pH-Wert von 1 bis 2 mit Salzsäure eingestellt. Der Niederschlag wird abfiltriert, mit Wasser gewaschen und sodann getrocknet.

### II. Beispiele für Haarfärbemittel

### Beispiel 4: Haarfärbemittel

| | |
|---|---|
| 0,61 g | 1,3-Bis(dicyanmethylen)indan |
| 10,00 g | Isopropanol |
| 1,00 g | Benzylalkohol |
| 10,00 g | Laurylalkoholdiglycolethersulfat-natriumsalz (28%ige wässerige Lösung) |
| ad 100,00 g | Wasser |

Das vorstehende gebrauchsfertige Haarfärbemittel wird unmittelbar vor Gebrauch durch Vermischen des Farbstoffes mit den restlichen Komponenten erhalten. Die Einstellung des pH-Wertes erfolgt entweder mit Milchsäure oder mit verdünnter Ammoniaklösung. Gebleichte Haare werden sodann 40 Minuten bei einer Temperatur von 40 °C mit dem Haarfärbemittel (einmal mit Milchsäure sauer und einmal mit verdünnter Ammoniaklösung basisch eingestellt) behandelt. Anschließend werden die Haare mit Wasser gespült und getrocknet. Das Haar erhält sowohl bei pH 5 als auch pH 8 eine intensive blaue Färbung.

### Beispiel 5: Haarfärbemittel

| | |
|---|---|
| 0,0015 g | 1,3-Bis(dicyanmethylen)indan |
| 0,0600 g | 5-Chlor-4-((2-hydroxyethyl)amino)-2-nitro-anilin |
| 0,0250 g | 1-((4-Amino-2-nitrophenyl)azo)-7-(trimethylammonium)- 2-naphthol-chlorid |
| 0,0300 g | 4-(Di(2-hydroxyethyl)amino)-2-nitro-anilin |
| 10,000 g | Ethanol |
| 10,000 g | Laurylalkoholdiglycolethersulfat-natriumsalz (28%ige wässerige Lösung) |
| ad 100,000 g | Wasser |

Das vorstehende gebrauchsfertige Haarfärbemittel wird unmittelbar vor Gebrauch durch Vermischen des Farbstoffes mit den restlichen Komponenten erhalten, wobei der pH-Wert der Färbelösung mit verdünnter Ammoniaklösung auf 8 eingestellt wird. Gebleichte Haare werden sodann 40 Minuten bei einer Temperatur von 40 °C mit den Haarfärbemittel behandelt. Anschließend werden die Haare mit Wasser gespült und getrocknet. Das Haar hat eine blonde Färbung erhalten.

### Beispiel 6: Haarfärbemittel

| | |
|---|---|
| 0,5200 g | 1,3-Bis(dicyanmethylen)indan |
| 0,2600 g | 4-(Ethyl(2-hydroxyethyl)amino)-N-(2-hydroxyethyl)-2-nitro-anilin-hydrochlorid |
| 0,0900 g | N-(2-Hydroxyethyl)-4-methyl-2-nitro-anilin |
| 0,0800 g | 5-Chlor-4-((2,3-dihydroxypropyl)amino)-2-nitro-anilin |
| 0,1700 g | 1-((4-Amino-2-nitrophenyl)azo)-7-(trimethylammonium)-2-naphthol-chlorid |
| 0,0160 g | Di(4-(diethylamino)phenyl)(4-(ethylamino)naphthyl)-carbenium-chlorid |
| 10,000 g | Ethanol |
| 10,000 g | Laurylalkoholdiglycolethersulfat-natriumsalz (28%ige wässerige Lösung) |
| ad 100,000 g | Wasser |

Das vorstehende gebrauchsfertige Haarfärbemittel wird unmittelbar vor Gebrauch durch Vermischen des Farbstoffes mit den restlichen Komponenten erhalten, wobei der pH-Wert der Färbelösung mit verdünnter Ammoniaklösung auf 8 eingestellt wird. Gebleichte Haare werden sodann 40 Minuten bei einer Temperatur von 40 °C mit den Haarfärbemittel behandelt. Anschließend werden die Haare mit Wasser gespült und getrocknet. Das Haar hat eine braune Färbung erhalten.

### Beispiel 7: Haarfärbemittel

| | |
|---|---|
| 0,0020 g | 1,3-Bis(dicyanmethylen)indan |
| 0,0005 g | Di-(4-aminophenyl)(4-amino-3-methylphenyl)-carbenium-chlorid |
| 10,000 g | Isopropanol |
| 1,000 g | Benzylalkohol |
| 10,000 g | Laurylalkoholdiglycolethersulfat-natriumsalz (28%ige wässerige Lösung) |
| ad 100,000 g | Wasser |

Das vorstehende gebrauchsfertige Haarfärbemittel wird unmittelbar vor Gebrauch durch Vermischen des Farbstoffes mit den restlichen Komponenten erhalten. Die Einstellung des pH-Wertes erfolgt entweder mit Milchsäure oder mit verdünnter Ammoniaklösung. Gebleichte Haare werden sodann 40 Minuten bei einer Temperatur von 40 °C mit den Haarfärbemittel behandelt. Anschließend werden die Haare mit Wasser gespült und getrocknet. Das Haar hat eine silbergraue Färbung erhalten.

### Beispiel 8: Haarfärbemittel

| | |
|---|---|
| 0,65 g | 2-Methyl-1 ,3-bis(dicyanmethylen)indan |
| 10,00 g | Isopropanol |
| 1,00 g | Benzylalkohol |
| 10,00 g | Laurylalkoholdiglycolethersulfat-natriumsalz (28%ige wässerige Lösung) |
| ad 100,00 g | Wasser |

Das vorstehende gebrauchsfertige Haarfärbemittel wird unmittelbar vor Gebrauch durch Vermischen des Farbstoffes mit den restlichen Komponenten erhalten. Die Einstellung des pH-Wertes erfolgt entweder mit Milchsäure oder mit verdünnter Ammoniaklösung. Gebleichte Haare werden sodann 40 Minuten bei einer Temperatur von 40 °C mit dem Haarfärbemittel (einmal mit Milchsäure sauer und einmal mit verdünnter Ammoniaklösung basisch eingestellt) behandelt. Anschließend werden die Haare mit Wasser gespült und getrocknet. Das Haar erhält sowohl bei pH 5 als auch pH 8 eine intensive blaue Färbung.

### Beispiel 9: Haarfärbemittel

| | |
|---|---|
| 0,80 g | 2-Phenyl-1,3-bis(dicyanmethylen)indan |
| 10,00 g | Isopropanol |
| 1,00 g | Benzylalkohol |
| 10,00 g | Laurylalkoholdiglycolethersulfat-natriumsalz (28%ige wässerige Lösung) |
| ad 100,00 g | Wasser |

Das vorstehende gebrauchsfertige Haarfärbemittel wird unmittelbar vor Gebrauch durch Vermischen des Farbstoffes mit den restlichen Komponenten erhalten. Die Einstellung des pH-Wertes erfolgt entweder mit Milchsäure oder mit verdünnter Ammoniaklösung. Gebleichte Haare werden sodann 40 Minuten bei einer Temperatur von 40 °C mit dem Haarfärbemittel (einmal mit Milchsäure sauer und einmal mit verdünnter Ammoniaklösung basisch eingestellt) behandelt. Anschließend werden die Haare mit Wasser gespült und getrocknet. Das Haar erhält sowohl bei pH 5 als auch pH 8 eine intensive blaue Färbung.

Alle in der vorliegenden Anmeldung enthaltenen Prozentangaben stellen, soweit nicht anders angegeben, Gewichtsprozente dar.

## Patentansprüche

1. Mittel zur Färbung von Haaren, **dadurch gekennzeichnet, daß** es (a) mindestens ein Indanderivat der Formel (I) oder dessen physiologisch verträgliches, wasserlösliches Salz, 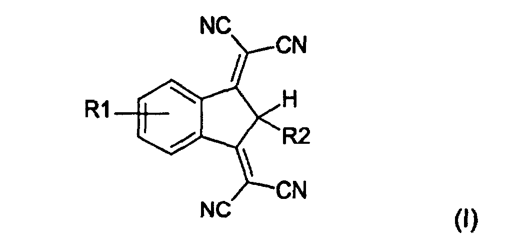 wobei die Reste R1und R2 unhabängig voneinander die folgende Bedeutung haben:
R1 = Wasserstoff, einem Halogenatom, einer Cyanogruppe, einer Hydroxygruppe, einer C₁-C₄-Alkoxygruppe, einer C₁-C₆-Alkylgruppe, einer Nitrogruppe, einer Aminogruppe, einer Alkylaminogruppe, einer Dialkylaminogruppe, einer -C(O)H-Gruppe, einer -COOR3 -Gruppe, einer SO₂R3 oder einer -C(O)CH₃-Gruppe (mit R3 jeweils gleich Wasserstoff, einer Phenylgruppe oder einer C₁-C₆-Alkygruppe);
R2 gleich Wasserstoff, einer C₁-C₆-Alkygruppe oder einer Phenylgruppe; sowie (b) für Haarfärbemittel übliche, kosmetisch verträgliche Zusätze enthält.

2. Mittel nach Anspruch 1 **dadurch gekennzeichnet, daß** die Verbindung der Formel (I) ausgewählt ist aus 1,3-Bis(dicyanmethylen)-indan, 2-Methyl-1,3-bis(dicyanmethylen)-indan und 2-Phenyl-1,3-bis(dicyanmethylen)-indan.

3. Mittel nach Anspruch 1 oder 2, dadurch gekennzeichet, daß die Verbindung der Formel (I) in einer Menge von 0,01 bis 10 Gewichtsprozent enthalten ist.

4. Mittel nach einem der Ansprüche 1 bis 3, **dadurch gekennzeichnet, daß** es zusätzlich mindestens einen direktziehenden Farbstoff aus der Gruppe bestehend aus Nitrofarbstoffen, Azofarbstoffen, Chinonfarbstoffen sowie basischen, sauren oder neutralen Farbstoffen enthält.

5. Mittel nach Anspruch 4, **dadurch gekennzeichnet, daß** die direktziehenden Farbstoffe in einer Menge von 0,01 bis 4 Gewichtsprozent enthalten sind.

6. Mittel nach einem der Ansprüche 1 bis 5, **dadurch gekennzeichnet, daß** es einen pH-Wert von 5,0 bis 8,0 aufweist.

7. Verwendung von Indanderivaten der Formel (1) oder deren physiologisch verträglichen, wasserlöslichen Salze, 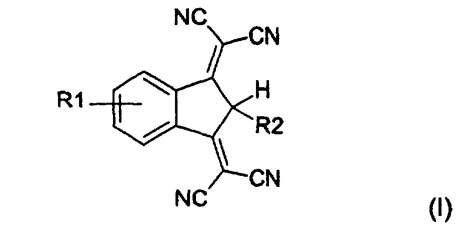 wobei die Reste R1und R2 unhabängig voneinander die folgende Bedeutung haben:
R1 = Wasserstoff, einem Hatogenatom, einer Cyanogruppe, einer Hydroxygruppe, einer C₁-C₄-Alkoxygruppe, einer C₁-C₆-Alkylgruppe, einer Nitrogruppe, einer Aminogruppe, einer Alkylaminogruppe, einer Dialkylaminogruppe, einer -C(O)H-Gruppe, einer -COOR3 -Gruppe, einer SO₂R3 oder einer -C(O)CH₃-Gruppe (mit R3 jeweils gleich Wasserstoff, einer Phenylgruppe oder einer C₁-C₆-Alkygruppe);
R2 gleich Wasserstoff, einer C₁-C₆-Alkygruppe oder einer Phenylgruppe; zur Färbung von Fasem.

8. Verwendung nach Anspruch 7, **dadurch gekennzeichnet, dass** die Verbindung der Formel (I) ausgewählt ist aus 1,3-Bis(dicyanmethylen)-indan, 2-Methyl-1,3-bis(dicyanmethylen)-indan und 2-Phenyl-1,3-bis(dicyanmethylen)-indan.

9. Verwendung nach Anspruch 7 oder 8, dadurch gekennzeichet, daß die Verbindung der Formel (l) in einer Menge von 0,01 bis 10 Gewichtsprozent eingesetzt wird.

10. Verwendung nach einem der Ansprüche 7 bis 9, dadurch gekennzeichet, daß die zu färbenden Fasem Haare sind.

## Claims

1. Composition for colouring hair, **characterized in that** it comprises (a) at least one indane derivative of the formula (I) or its physiologically compatible, water-soluble salt, 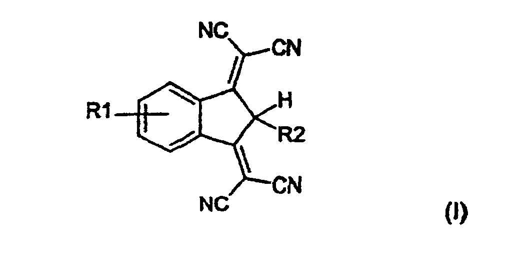 where the radicals R1 and R2, independently of one another, have the following meanings:
R1 = hydrogen, a halogen atom, a cyano group, a hydroxyl group, a C₁-C₄-alkoxy group, a C₁-C₆-alkyl group, a nitro group, an amino group, an alkylamino group, a dialkylamino group, a -C(O)H group, a -COOR3 group, an SO₂R3 or a -C(O)CH₃ group (where R3 is in each case hydrogen, a phenyl group or a C₁-C₆-alkyl group);
R2 is hydrogen, a C₁-C₆-alkyl group or a phenyl group; and (b) cosmetically compatible additives customary for hair colorants.

2. Composition according to Claim 1, **characterized in that** the compound of the formula (I) is chosen from 1,3-bis (dicyanomethylene)indane, 2-methyl-1, 3-bis (dicyanomethylene) indane and 2-phenyl-1, 3-bis(dicyanomethylene)indane.

3. Composition according to Claim 1 or 2, **characterized in that** the compound of the formula (I) is present in an amount of from 0.01 to 10 per cent by weight.

4. Composition according to one of Claims 1 to 3, **characterized in that** it additionally comprises at least one direct dye from the group consisting of nitro dyes, azo dyes, quinone dyes and basic, acidic or neutral dyes.

5. Composition according to Claim 4, **characterized in that** the direct dyes are present in an amount of from 0.01 to 4 per cent by weight.

6. Composition according to one of Claims 1 to 5, **characterized in that** it has a pH of from 5.0 to 8.0.

7. Use of indane derivatives of the formula (I) or physiologically compatible, water-soluble salts thereof, 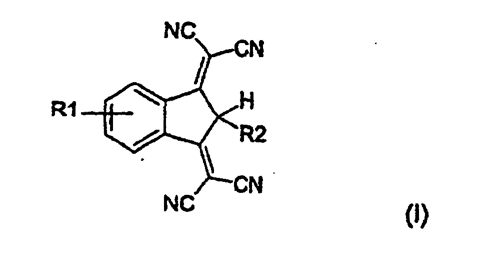 where the radicals R1 and R2, independently of one another, have the following meanings:
R1 = hydrogen, a halogen atom, a cyano group, a hydroxyl group, a C₁-C₄-alkoxy group, a C₁-C₆-alkyl group, a nitro group, an amino group, an alkylamino group, a dialkylamino group, a -C(O)H group, a -COOR3 group, an SO₂R3 or a -C(O)CH₃ group (where R3 is in each case hydrogen, a phenyl group or a C₁-C₆-alkyl group);
R2 is hydrogen, a C₁-C₆-alkyl group or a phenyl group;
for colouring fibres.

8. Use according to Claim 7, **characterized in that** the compound of the formula (I) is chosen from 1,3-bis(dicyanomethylene)indane, 2-methyl-1,3-bis-(dicyanomethylene)indane and 2-phenyl-1,3-bis-(dicyanomethylene)indane.

9. Use according to Claim 7 or 8, **characterized in that** the compound of the formula (I) is used in an amount of from 0.01 to 10 per cent by weight.

10. Use according to one of Claims 7 to 9, **characterized in that** the fibres to be coloured are hairs.

## Revendications

1. Produit pour la teinture des cheveux, **caractérisé en ce qu'**il contient (a) au moins un dérivé d'indane de formule (I) ou un sel hydrosoluble, physiologiquement acceptable, d'un tel dérivé, 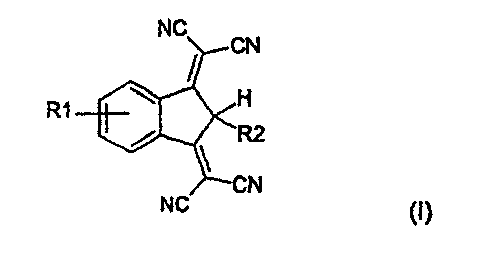 formule dans laquelle les radicaux R1 et R2 ont, indépendamment l'un de l'autre, les significations suivantes :
R1 représente un atome d'hydrogène ou d'halogène, un groupe cyano, un groupe hydroxy, un groupe alcoxy en C₁-C₄, un groupe alkyle en C₁-C₆, un groupe nitro, un groupe amino, un groupe alkylamino, un groupe dialkylamino, un groupe -C(O)H, un groupe -COOR3, un groupe SO₂R3 ou un groupe -C(O)CH₃ (où R3 représente un atome d'hydrogène, un groupe phényle ou un groupe alkyle en C₁-C₆);
R2 représente un atome d'hydrogène, un groupe alkyle en C₁-C₆ ou un groupe phényle ;
ainsi que (b) des additifs cosmétiquement acceptables, usuels dans des produits de teinture pour cheveux.

2. Produit selon la revendication 1, **caractérisé en ce que** le composé de formule (I) est choisi parmi le 1,3-bis(dicyanométhylène)indane, le 2-méthyl-1,3-bis(dicyanométhylène)indane et le 2-phényl-1,3-bis(dicyanométhylène)indane.

3. Produit selon la revendication 1 ou 2, **caractérisé en ce que** le composé de formule (I) est contenu en une quantité de 0,01 à 10 % en poids.

4. Produit selon l'une quelconque des revendications 1 à 3, **caractérisé en ce qu'**en outre il contient au moins un colorant direct choisi dans le groupe constitué par les colorants nitrés, les colorants azoïques, les colorants quinoniques ainsi que les colorants basiques, acides ou neutres.

5. Produit selon la revendication 4, **caractérisé en ce que** les colorants directs sont contenus en une quantité de 0,01 à 4 % en poids.

6. Produit selon l'une quelconque des revendications 1 à 5, **caractérisé en ce qu'**il présente un pH de 5,0 à 8,0.

7. Utilisation de dérivés d'indane de formule (I) ou de sels hydrosolubles, physiologiquement acceptables, de tels dérivés, 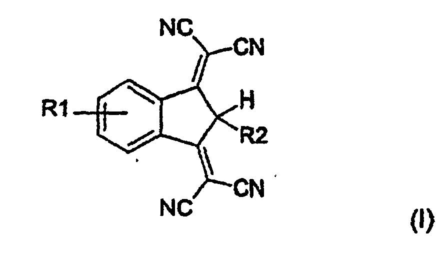 formule dans laquelle les radicaux R1 et R2 ont, indépendamment l'un de l'autre, les significations suivantes :
R1 représente un atome d'hydrogène ou d'halogène, un groupe cyano, un groupe hydroxy, un groupe alcoxy en C₁-C₄, un groupe alkyle en C₁-C₆, un groupe nitro, un groupe amino, un groupe alkylamino, un groupe dialkylamino, un groupe -C(O)H, un groupe -COOR3, un groupe SO₂R3 ou un groupe -C(O)CH₃ (où R3 représente un atome d'hydrogène, un groupe phényle ou un groupe alkyle en C₁-C₆)
R2 représente un atome d'hydrogène, un groupe alkyle en C₁-C₆ ou un groupe phényle ;
pour la teinture de fibres.

8. Utilisation selon la revendication 7, **caractérisée en ce que** le composé de formule (I) est choisi parmi le 1,3-bis(dicyanométhylène)indane, le 2-méthyl-1,3-bis(dicyanométhylène)indane et le 2-phényl-1,3-bis(dicyanométhylène)indane.

9. Utilisation selon la revendication 7 ou 8, **caractérisée en ce que** le composé de formule (I) est utilisé en une quantité de 0,01 à 10 % en poids.

10. Utilisation selon l'une quelconque des revendications 7 à 9, **caractérisée en ce que** les fibres à teindre sont des cheveux.
